(19) Europäisches Patentamt
European Patent Office
Office européen des brevets

(11) Veröffentlichungsnummer: **0 146 797 B1**

(12) **EUROPÄISCHE PATENTSCHRIFT**

(45) Veröffentlichungstag der Patentschrift: **11.09.91**

(51) Int. Cl.5: **C07D 413/04**, C07D 417/04, C07D 409/04, C07D 413/14, C07D 333/68

(21) Anmeldenummer: **84114308.4**

(22) Anmeldetag: **27.11.84**

Teilanmeldung 90107116.7 eingereicht am 27/11/84.

# (54) 2-Aminothiophenverbindungen.

(30) Priorität: **07.12.83 DE 3344294**

(43) Veröffentlichungstag der Anmeldung:
**03.07.85 Patentblatt 85/27**

(45) Bekanntmachung des Hinweises auf die Patenterteilung:
**11.09.91 Patentblatt 91/37**

(84) Benannte Vertragsstaaten:
**CH DE FR GB LI**

(56) Entgegenhaltungen:
**DE-A- 2 553 621**
**FR-A- 2 128 234**
**US-A- 3 963 750**
**US-A- 3 989 505**

(73) Patentinhaber: **BAYER AG**
**W-5090 Leverkusen 1 Bayerwerk(DE)**

(72) Erfinder: **Fürstenwerth, Hauke, Dr.**
**Morgengraben 3**
**W-5000 Köln 80(DE)**

**EP 0 146 797 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind 2-Aminothiophene der Formel

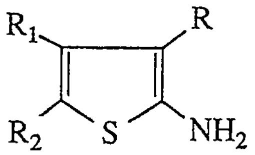

(I)

worin

- R CN, $C_1$-$C_4$-Alkoxycarbonyl, $CONH_2$, CONH-$C_1$-$C_4$-Alkyl oder CON($C_1$-$C_4$-Alkyl)$_2$,
- $R_1$ H, $C_1$-$C_4$-Alkyl, Benzyl, Phenyl oder Tolyl und
- $R_2$ Pyridyl-2(-3 oder -4), Pyrimidyl-2(oder -4), Pyrazinyl, Pyridazolyl-3(oder -4), Thiazolyl-2(oder -4), Benzthiazolyl-2, Oxazolyl-2, Benzoxazolyl-2, 1,3,4-Oxdiazolyl-2, 1,2,4-Triazolyl-3, Imdazolyl-2, Benzimidazolyl-2 oder 1,2,4-Oxidazolyl-3(oder -5), wobei die monocyclischen Reste durch Methyl oder Phenyl und die bicyclischen Reste im Benzolkern durch Methyl, Cl, Br, $NO_2$ oder $C_1$-$C_4$-Alkylsulfonyl weiter substituiert sein können, bedeuten.

Ganz besonders bevorzugt sind Verbindungen der Formel I, worin R CN bedeutet.

Unter diesen sind wieder solche Typen der angegebenen Formeln bevorzugt, worin

$R_1$ = H oder Methyl und

$R_2$ = einen 1,2,4-Oxadiazolylrest bedeuten.

Die neuen Verbindungen sind nach verschiedenen Verfahren zugänglich.

Ein allgemein anwendbares Verfahren zur Herstellung der Verbindungen der Formel I ist dadurch gekennzeichnet, daß man Verbindungen, die in einer ihrer tautomeren Formen der Formel

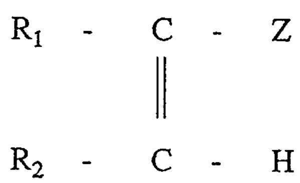

(II)

entsprechen,

worin

$R_1$ und $R_2$ die obengenannte Bedeutung haben und

Z für eine Hydroxyl-, Ether oder Amingruppe steht,

mit Verbindungen der Formel

R - $CH_2$ - CN (III)

in Gegenwart von elementarem Schwefel unter den Bedingungen der bekannten Knoevenagel-Kondensation umsetzt.

Die Verbindungen der Formeln II - III sind entweder bekannt oder nach an sich bekannten Methoden leicht herstellbar.

Auch die Reaktionsbedingungen der durch die Gegenwart des Schwefels gekennzeichneten Umsetzungen sind an sich bekannt.

In diesem Zusammenhang wird z.B. auf die folgende Literatur verwiesen.

Bücher: H.D. Hartough "Thiophene and Its Derivatives" und "Compounds with Condensed Thiophene Rings", beide aus der Serie "The Chemistry of Heterocyclic Compounds", Interscience Publishers Inc., New York 1952.

Die Arbeiten von Gewald (et al.) Z.Chem. 7 (1967), Heft 5, 186; Chem. Berichte 101 (1068), 1933; Chem. Berichte 98, (1965), 3571; Z.Chem. 2, (1962), 305. D.E.Wolf et al. "The Preparation of Thiophenes and Tetrahydrothiophenes", Organic Reactions 6, (1951), 410 - 468.

Als organische Basen kommen im wesentlichen primäre, sekundäre und vorzugsweise tertiäre Amine in

Betracht. Die Auswahl der Basen kann sich nach wirtschaftlichen Gesichtspunkten richten, da vom Effekt her gesehen innerhalb der Gruppen primärer, sekundärer und tertiärer Amine keine Unterschiede zu beobachten sind. Bevorzugt sind daher beispielsweise Trimethyl-, Triethyl-, Tripropyl-, Dimethylethyl-, Diethylmethylamin oder Dimethylethanolamin.

Als Lösungsmittel für die Reaktion eignen sich insbesondere Alkohole, Glykole, Glykolether oder Ether. Im einzelnen seien beispielsweise Methanol, Ethanol, Propanol, Glykol, Methyl-, Ethyl- oder Butylglykol sowie Tetrahydrofuran oder Dioxan genannt.

Einzelheiten der Reaktionsführung können den Beispielen entnommen werden, in denen sich Angaben über Teile und Prozente, sofern nicht anders vermerkt, auf das Gewicht beziehen.

Die neuen Verbindungen der Formel I sind wertvolle Zwischenprodukte zur Herstellung von Wirkstoffen auf dem Gebiet der Pharmazie und des Pflanzenschutzes, indem man diese Verbindungen beispielsweise mit Isocyanaten zu den entsprechenden Harnstoffen umsetzt.

Ganz besonders geeignet sind jedoch die neuen Aminothiophene als Diazokomponenten zum Aufbau von Azofarbstoffen, beispielsweise zur Herstellung von nebendichtearmen blaugrünen Azofarbstoffkörpern für das fotografische Farbdiffusionsverfahren (vgl. z.B. US-PS 4 346 161).

Die Umsetzung der Verbindungen der Formeln II mit III in Gegenwart von Schwefel, wird vorteilhaft unter Verwendung von nahezu stöchiometrischen Mengen der Reaktionsteilnehmer im organischen inerten Medium, gegebenenfalls in Gegenwart eines Katalysators durchgeführt.

Zweckmäßigerweise nimmt man die Umsetzung so vor, daß man die Verbindungen der Formeln II und III sowie den Schwefel in feinverteilter Form in einem Lösungsmittel vorlegt und dann eine organische Base bei möglichst niedriger Temperatur, zutropft. Anschließend wird die Temperatur erhöht und vorzugsweise zwischen 30° und Siedetemperatur (Rückflußtemperatur) gehalten.

Nach beendeter Umsetzung kann dann das Reaktionsprodukt nach an sich üblichen Methoden isoliert werden, z.B. durch Filtrieren, gegebenenfalls Waschen und/oder Umkristallisieren.

In DE-A 25 53 621, US-A 3 963 750 und US-A 3 989 505 sind bereits 2-Amino-thiophenverbindungen ähnlicher Struktur beschrieben, die jedoch in der 5-Stellung keinen Oxdiazol- oder Thiazolrest aufweisen.

Beispiel 1

20 Teile 3-Phenyl-5-acetonyl-1,2,4-oxadiazol und 7 Teile Malodinitril und 3,2 Teile Schwefel werden in 80 Teilen Ethanol vorgelegt. Unter Kühlung mit einem Wasserbad werden 20 Teile Triethylamin zugetropft. Man läßt 1 Stunde bei Raumtemperatur nachrühren und kocht dann 2 Stunden am Rückfluß. Nach Abkühlen auf Raumtemperatur werden die ausgefallenen Kristalle isoliert, mit Ethanol gewaschen und im Vakuum getrocknet. Man erhält 23 Teile Produkt der Formel

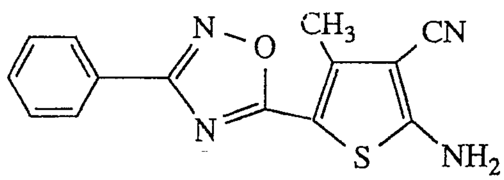

Fp: 275°C

Analog erhält man die Verbindung der Formel

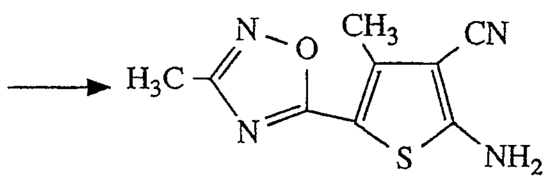

Beispiel 2

10 Teile 3-Phenyl-5-acetonyl-1,2,4-oxadiazol und 6 Teile Cyanessigsäureethylester und 1,6 Teile Schwefel werden in 80 Teile Ethanol vorgelegt. Unter Kühlung gibt man 10 Teile Diethylamin zu und läßt 1

h nachrühren. Dann wird das Reaktionsgemisch 2 h am Rückfluß gekocht. Nach dem Abkühlen wird das kristalline Produkt isoliert, mit Ethanol gewaschen und getrocknet. Man erhält 8 Teile Produkt der Formel

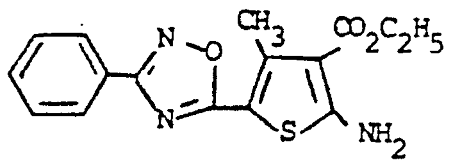

Fp: 175°C

Analog lassen sich die in den Beispielen 2 - 10 eingesetzten Acetonyloxadiazole in die entsprechenden 3-Carbonester-thiophene überführen.

Beispiel 3

117 Teile β-Amino-thiocrotonsäureamid werden in 250 Teilen Ethanol mit 95 Teilen Chloraceton 30 Minuten am Rückfluß gekocht. Danach wird auf Raumtemperatur abgekühlt. Sodann werden 32 Teile Schwefel und 66 Teile Malodinitril zugegeben und unter Kühlung 200 Teile Triethylamin zugetropft. Dann wird das Reaktionsgemisch 2 h am Rückfluß gekocht. Nach Abkühlen auf Raumtemperatur erhält man nach Isolieren, Waschen mit Ethanol und Trocknen 150 Teile Produkt der Formel

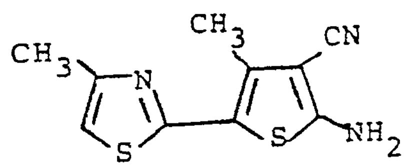

Fp: 250°C

Beispiel 4

Es wird wie in Beispiel 3 gearbeitet jedoch werden statt Malodinitril 120 Teile Cyanessigsäureethylester eingesetzt. Man erhält 120 Teile Produkt der Formel

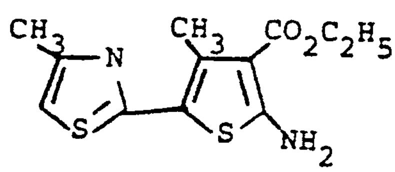

Fp: 150°C

Arbeitet man nach den Angaben der Beispiele 12 und 13 setzt jedoch anstelle von Chloraceton die in folgender Tabelle aufgeführten Halogenketone ein, so erhält man die entsprechenden Thiophene

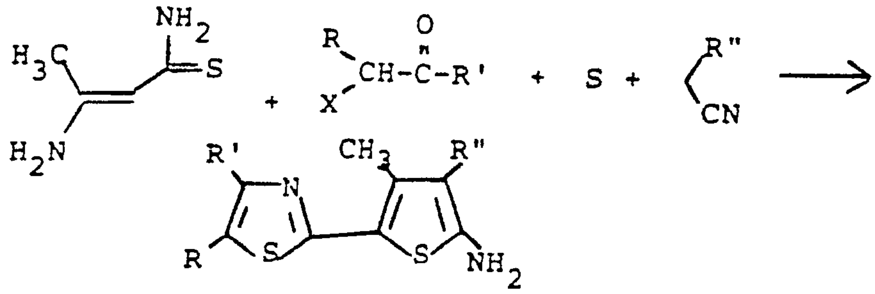

| Beispiel Nr. | R | R' | X | R" |
|---|---|---|---|---|
| 5 | H | H | Cl | CN |
| 6 | $CH_3$ | $CH_3$ | Cl | CN |
| 7 | $CH_3$ | $CH_3$ | Cl | $CO_2C_2H_5$ |
| 8 | H | $C_6H_5$ | Br | CN |
| 9 | H | $C_6H_5$ | Br | $CO_2C_2H_5$ |

Die Thiophene der Beispiele 3 - 9 können auch hergestellt werden, indem man nach den Angaben der Beispiele 1 und 2 entsprechende Acetonylthiazole anstelle des Phenyl-acetonyl-oxadiazols mit Schwefel und Malodinitril oder Cyanessigester.

**Patentansprüche**

1. Aminothiophene der Formel

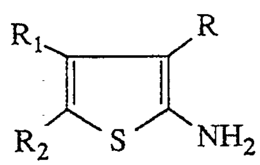

(I)

worin

R CN, $C_1$-$C_4$-Alkoxycarbonyl, $CONH_2$, CONH-$C_1$-$C_4$-Alkyl oder CON($C_1$-$C_4$-Alkyl)$_2$,
$R_1$ H, $C_1$-$C_4$-Alkyl, Benzyl, Phenyl oder Tolyl und
$R_2$ Pyridyl-2(-3 oder -4), Pyrimidyl-2(oder -4), Pyrazinyl, Pyridazolyl-3(oder -4), Thiazolyl-2(oder -4), Benzthiazolyl-2, Oxazolyl-2, Benzoxazolyl-2, 1,3,4-Oxdiazolyl-2, 1,2,4-Triazolyl-3, Imdazolyl-2, Benzimidazolyl-2 oder 1,2,4-Oxidazolyl-3(oder -5), wobei die monocyclischen Reste durch Methyl oder Phenyl und die bicyclischen Reste im Benzolkern durch Methyl, Cl, Br, $NO_2$ oder $C_1$-$C_4$-Alkylsulfonyl weiter substituiert sein können, bedeuten.

2. Aminthiophen der Formel

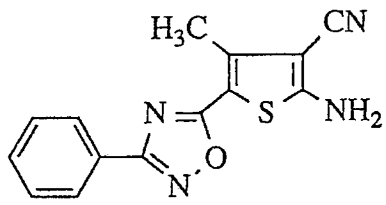

3. Verwendung der Aminothiophene gemäß Anspruch 1 als Diazokomponenten zum Aufbau von Azofarbstoffen.

**Claims**

1. Aminothiophenes of the formula

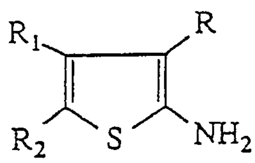

(I)

wherein

R denotes CN, $C_1$-$C_4$-alkoxycarbonyl, $CONH_2$, CONH-$C_1$-$C_4$-alkyl or CON($C_1$-$C_4$-alkyl)$_2$,
$R_1$ denotes H, $C_1$-$C_4$-alkyl, benzyl, phenyl or tolyl and
$R_2$ denotes pyrid-2(-3 or -4)-yl, pyrimid-2(or -4)-yl, pyrazinyl, pyridazol-3(or -4)-yl, thiazol-2(or -4)-yl, benzothiazol-2-yl, oxazol-2-yl, benzoxazol-2-yl, 1,3,4-oxadiazol-2-yl, 1,2,4-triazol-3-yl, imidazol-2-yl, benzimidazol-2-yl or 1,2,4-oxidazol-3(or -5)-yl, it being possible for the monocyclic radicals to be further substuituted by methyl or phenyl and the bicyclic radicals to be further substituted in the benzene nucleus by methyl, Cl, Br, $NO_2$ or $C_1$-$C_4$-alkylsulphonyl.

2. Aminothiophene of the formula

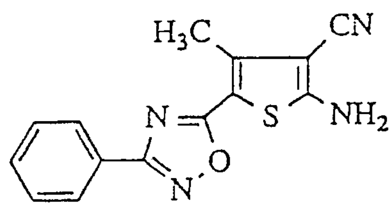

3. Use of the aminothiophenes according to Claim 1 as diazo components for building up azo dye-stuffs.1

**Revendications**

1. Aminothiophènes de formule

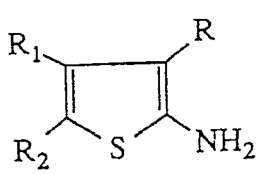

(I)

dans laquelle

R représente un groupe CN, (alkoxy en $C_1$ à $C_4$)carbonyle, $CONH_2$, CONH-(alkyle en $C_1$ à $C_4$) ou CON(alkyle en $C_1$ à $C_4$)$_2$,

$R_1$ représente H, un groupe alkyle en $C_1$ à $C_4$, benzyle, phényle ou tolyle et

$R_2$ représente un groupe pyridyl-2(-3 ou -4), pyrimidyl-2(ou -4), pyrazinyle, pyridazolyl-3-(ou -4), thiazolyl-2(ou -4), benzothiazolyl-2, oxazolyl-2, benzoxazolyl-2, 1,3,4-oxodiazolyl-2, 1,2,4-triazolyl-3, imidazolyl-2, benzimidazolyl-2 ou 1,2,4-oxodiazolyl-3(ou -5), les restes monocycliques pouvant être substitués en outre par des radicaux méthyle ou phényle et les restes bicycliques pouvant être substitués en outre dans le noyau benzénique par des radicaux méthyle, Cl, Br, $NO_2$ ou alkylsulfonyle en $C_1$ à $C_4$.

**2.** Aminothiophène de formule

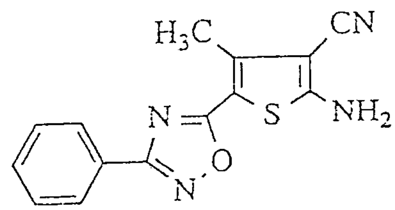

**3.** Utilisation des aminothiophènes suivant la revendication 1 comme composants diazotables pour la synthèse de colorants azoïques.